# EUROPÄISCHE PATENTSCHRIFT

(11) **EP 1 866 320 B1**
(45) Veröffentlichungstag und Bekanntmachung des Hinweises auf die Patenterteilung: **18.02.2009**
(21) Anmeldenummer: 05810910.9
(22) Anmeldetag: 03.11.2005
(51) Int. Cl.: C07F 9/6571, C09K 21/12, C07D 251/34

(54) **STICKSTOFFHALTIGE VERBRÜCKTE DERIVATE VON 6H-DIBENZ(E,E)(1,2)-OXAPHOSPHORIN-6-OXIDEN, VERFAHREN ZU IHRER HERSTELLUNG SOWIE IHRE VERWENDUNG ALS FLAMMSCHUTZMITTEL**
NITROGEN-CONTAINING BRIDGED DERIVATIVES OF 6H-DIBENZ(E,E)(1,2)-OXAPHOSPHORINE-6-OXIDES METHOD FOR PRODUCTION AND USE THEREOF AS FLAME-RETARDANT AGENTS
DERIVES PONTES AZOTES D'OXYDES DE 6H-DIBENZ(E,E)(1,2)-OXAPHOSPHORINE-6-OXIDES, PROCEDE POUR LEUR PRODUCTION ET LEUR UTILISATION COMME AGENTS IGNIFUGES

(30) Priorität: 09.02.2005 DE 102005005879
(43) Veröffentlichungstag der Anmeldung: 19.12.2007
(73) Patentinhaber: Schill + Seilacher "Struktol" Aktiengesellschaft, 22113 Hamburg (DE)
(72) Erfinder: JUST, Berthold, 22397 Hamburg (DE); DITTRICH, Uwe, 01445 Radebeul (DE); KELLER, Holger, 71069 Sindelfingen-Darmsheim (DE); DÖRING, Manfred, 76744 Wörth-Büchelberg (DE); STORZER, Uwe, 72076 Tüblingen (DE); CIESIELSKI, Michael, 06217 Merseburg (DE)
(74) Vertreter: Bunke, Holger
(86) Internationale Anmeldenummer: PCT/EP2005/011785
(87) Internationale Veröffentlichungsnummer: WO 2006/084488

(56) Entgegenhaltungen:
- US-A1- 2005 038 279
- PATENT ABSTRACTS OF JAPAN Bd. 2000, Nr. 23, 10. Februar 2001 (2001-02-10) & JP 2001 172555 A (SUMITOMO DUREZ CO LTD), 26. Juni 2001 (2001-06-26)
- PATENT ABSTRACTS OF JAPAN Bd. 2002, Nr. 03, 3. April 2002 (2002-04-03) & JP 2001 323268 A (SANKO KK; SAITO KASEIHIN KENKYUSHO:KK), 22. November 2001 (2001-11-22)
- PATENT ABSTRACTS OF JAPAN Bd. 010, Nr. 366 (C-390), 6. Dezember 1986 (1986-12-06) & JP 61 162541 A (ASAHI CHEM IND CO LTD), 23. Juli 1986 (1986-07-23)

## Beschreibung

Die Erfindung betrifft stickstoffhaltige verbrückte Derivate von 6H-Di-benz[c,e][1,2]-oxaphosphorin-6-oxiden, ein Verfahren zur ihrer Herstellung sowie ihre Verwendung als Flammschutzmittel für Polymere und daraus hergestellte Erzeugnisse.

Dibenz[c,e][c,2]-oxaphosphorin-6-oxid, auch "DOP" genannt, und seine Derivate sind seit langem als wirksame Flammschutzmittel für Polymere bekannt (DT 2034887 C3, DT 2646218 A1, DE 195 22876 C1).

Aus der Literatur sind Aminoderivate von Arylphosphanoxiden (J. Appl. Polym. Sci. 1997, 63, 895), Phosphorsäurearylestem (J. Polym. Sci. A: Polym. Chem. 1997, 35, 565), und Phosphazenen (J. Appl. Polym. Sci. 1998, 67, Seite 461 / Progr. Polym. Sci. 2002, 27, Seite 1680) als reaktive Flammschutzmittel bekannt (Polymer Degr. Stab. 1998, 60, 169).

Aufgrund der ausgewiesenen Flammschutzwirkung der Dibenz[c,e][1,2]-oxaphosphorin-6-oxide wurden auch schon Aminoderivate dieser Verbindungen untersucht, beispielsweise eine Reihe unterschiedlicher 6-Aminomethyl-Derivate (JP 2003-105058, JP 2002-284850, JP 2001-323268, US 4742088), die allesamt durch Aminomethylierung des 6H-Dibenz[c,e][1,2]-oxaphosphorin-6-oxids mittels Formaldehyd und primärer oder sekundärer Amine erzeugt wurden. Der Nachteil dieser 6-Aminomethyl-Derivate besteht darin, daß in Gegenwart von Wasser, die Rückreaktion zu den Edukten unter Freisetzung der 6H-Dibenz[c,e][1,2]-oxaphosphorin-6-oxide abläuft. Gerade bei höheren Temperaturen, wie sie bei der Einarbeitung von Flammschutzmitteln in Thermoplaste angewendet werden, läuft die Rückreaktion bevorzugt ab. Die unerwünschte Freisetzung der 6H-Dibenz[c,e][1,2]-oxaphosphorin-6-oxide führt dann zu einem sauren Abbau vieler thermoplastischer Polymere.

Es wurden jedoch bisher keine stickstoffhaltigen verbrückten Derivate von 6H-Dibenz[c,e][1,2]-oxaphosphorin-6-oxiden beschrieben, die mehr als ein Brückenkohlenstoffatom zwischen dem Phosphoratom des Phospha-oxaphenanthren-Ringsystems und dem Stickstoffatom der Brücke aufweisen. Ferner wurden bisher keine polymeren stickstoffhaltigen verbrückten Derivate von 6H-Dibenz[c,e][1,2]-oxaphosphorin-6-oxiden beschrieben.

Der vorliegenden Erfindung liegt somit die Aufgabe zugrunde, stickstoffhaltige verbrückte Derivate von 6H-Dibenz[c,e][1,2]-oxaphosphorin-6-oxiden bereitzustellen und ein Verfahren zu ihrer Herstellung sowie ihre Verwendung anzugeben. Es ist insbesondere ein Ziel der Erfindung, ein Verfahren zur Verfügung zu stellen, das die o.g. Nachteile des Standes der Technik vermeidet. Insbesondere soll das Verfahren von kommerziell leicht erhältlichem DOP bzw. Derivaten desselben ausgehen und einen möglichst einfachen und kostengünstigen sowie möglichst halogenfreien Syntheseweg liefern.

Diese Aufgabe wird erfindungsgemäß gelöst durch die Verfahren zur Herstellung der Verbindungen gemäß den Patentansprüchen 1 bis 13, die Verbindungen der Patentansprüche 14 bis 17, die den Formeln I und II entsprechen sowie deren Verwendung als Flammschutzmittel für Polymere und daraus hergestellte Erzeugnisse. Bevorzugte Ausführungsformen ergeben sich aus den abhängigen Ansprüchen.

Die vorliegende Erfindung liefert ein Verfahren zur Herstellung von stickstoffhaltigen verbrückten Derivaten von 6H-Dibenz[c,e][1,2]-oxaphosphorin-6-oxiden der Formeln (I) und (II) 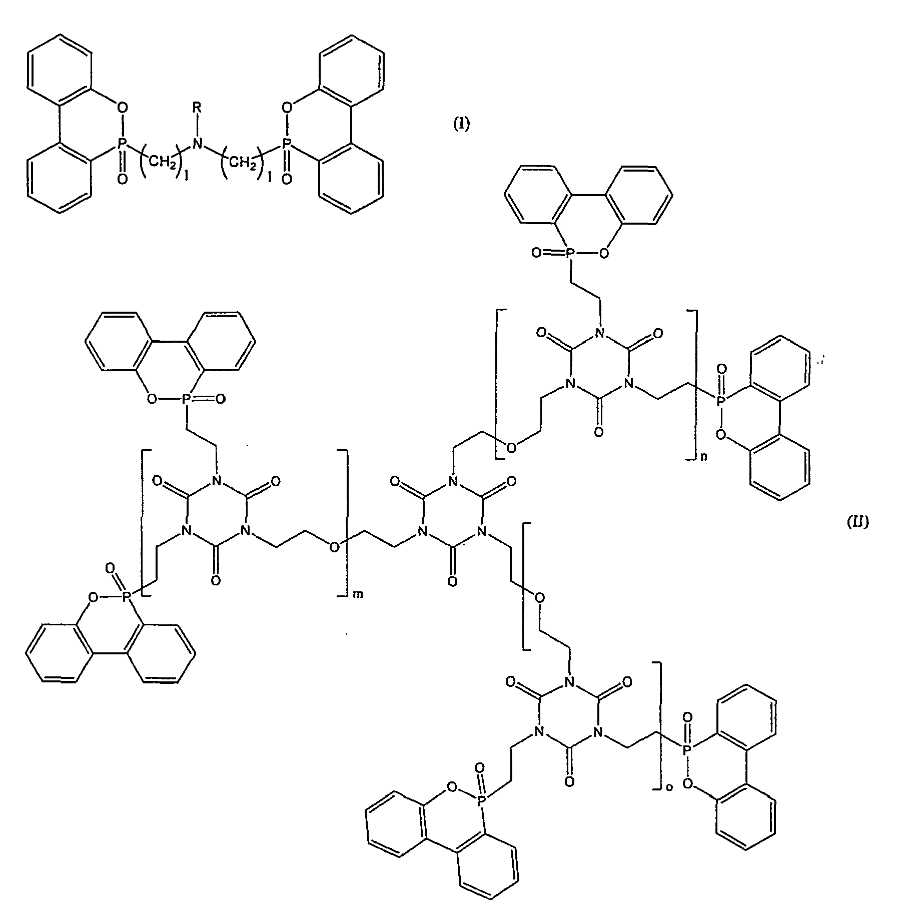 worin R einer der folgenden Reste:

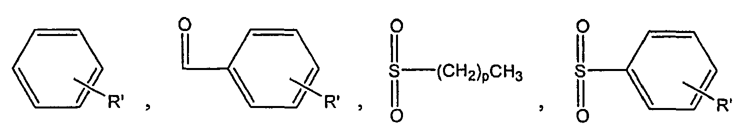

1 eine ganze Zahl von 2 bis 10, m und p ganze Zahlen von 1 bis 20, n und o ganze Zahlen von 0 bis 20 und R' = Wasserstoff oder Alkyl bedeuten, bei dem:
(a) ein 6-Alkoxy-[6H]-dibenz[c,e][1,2]-oxaphosphorin mit einem Bishydroxyalkylamin oder einem mehrwertigen Alkohol, der durch Polykondensation von 1,3,5-Tris(2-hydroxyethyl)cyanursäure gebildet wurde, unter Bildung eines Zwischenprodukts umgesetzt wird und
(b) das Zwischenprodukt aus (a) durch Zugabe katalytischer Mengen Alkylierungsmittel in ein stickstoffhaltiges verbrücktes 6H-Dibenz[c,e][1,2]-oxaphosphorin-6-oxid-Derivat der Formel 1 oder II übergeführt wird.

Soweit in dieser Anmeldung von mehrwertigen Molekülresten die Rede ist, ist damit die Bindungswertigkeit dieser Molekülreste gemeint. In bezug auf Alkohole bedeutet Wertigkeit die Zahl der OH-Gruppen des Alkohols.

Die Verwendung mehrwertiger Alkohole bei der vorliegenden Erfindung ermöglicht die Darstellung von mehrwertigen ("verbrückten") Derivaten, die bei der anschließenden Verwendung als Flammschutzmittel in polymeren Netzwerken vorteilhafter als nicht-verbrückte Derivate in die Polymere eingebunden werden können und eine verbesserte Flammschutzwirkung aufweisen können.

Zur Herstellung der stickstoffhaltigen verbrückten 6H-Dibenz[c,e][1,2]-oxaphosphorin-6-oxide **I** und **II** werden zunächst in einem ersten Schritt (a) ein 6-Alkoxy-[6H]-dibenz[c,e][1,2]-oxaphosphorin mit einem Bishydroxyalkylamin **III** oder einem mehrwertigen Alkohol **IV**, der durch Polykondensation von 1,3,5-Tris(2-hydroxyethyl)cyanursäure gebildet wurde, wobei das Bishydroxyalkylamin **III** und der mehrwertige Alkohol **IV** die nachfolgenden Formeln aufweisen, zu einem Zwischenprodukt umgesetzt.

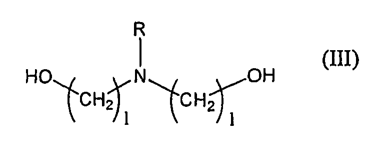

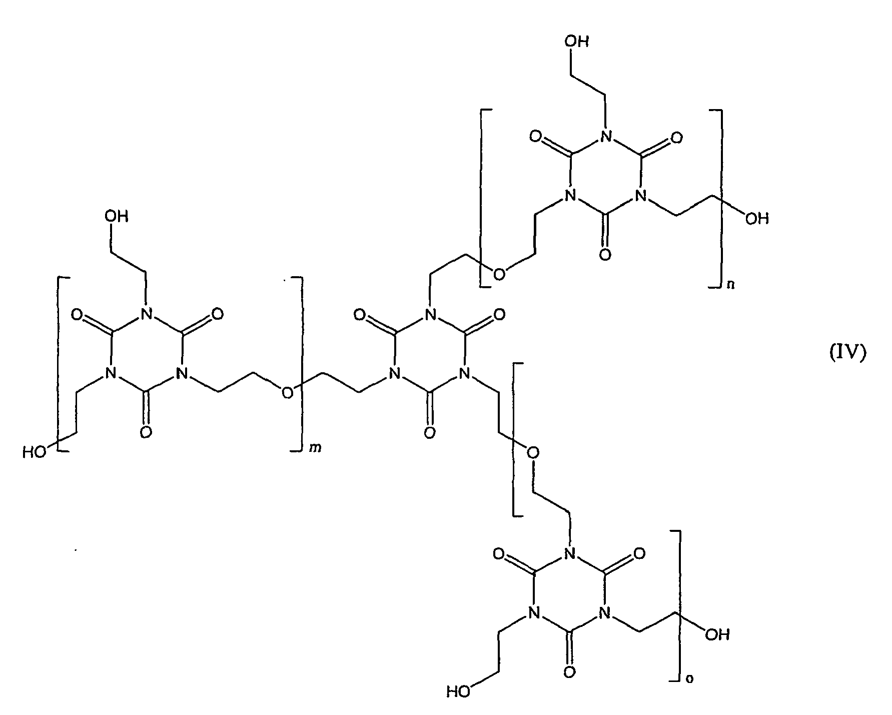

Als Edukt in Stufe (a) des erfindungsgemäßen Verfahrens wird ein 6-Alkoxy[6H]-dibenz[c,e][1,2]-oxaphosphorin verwendet, das nach einem in DE 102 06 982 A1 offenbarten Verfahren hergestellt wurde. Ausgehend von 6H-Dibenz[c,e][1,2]-oxaphosphorin-6-oxid werden die 6-Alkoxy-(6H)-dibenz[c,e][1,2]-oxaphosphorine durch säurekatalysierte Umsetzung mit Orthocarbonsäureestern und geeigneten Alkoholen erhalten. Bevorzugt wird 6-Ethoxy[6H]-dibenz[c,e][1,2]-oxaphosphorin als Edukt verwendet.

Als Bishydroxyalkylamine eignen sich Verbindungen der Formel **III**, worin R einer der folgenden Reste: 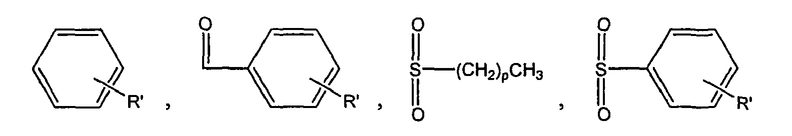 insbesondere eine Phenyl- oder p-Toluolsulfonylgruppe, 1 eine ganze Zahl von 2 bis 10, p eine ganze Zahl von 1 bis 20 und R' Wasserstoff oder Alkyl bedeuten.

Beispiele für Bishydroxyalkylamine sind z.B. Bis(2-hydroxyethyl)p-toluolsulfonylamin, sowie Bis(2-hydroxyethyl)phenylamin.

Als mehrwertige Alkohole eignen sich Verbindungen der Formel **IV,** worin m eine ganze Zahl von 1 bis 20 ist, und n und o ganze Zahlen von 0 bis 20 sind. Insbesondere eignen sich mehrwertige Alkohole der Formel **IVa**, 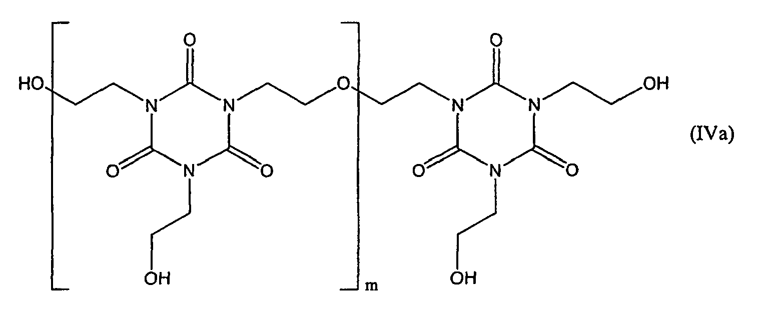 die sich aus mehrwertigen Alkoholen der Formel **IV** ableiten, bei denen m eine ganze Zahl von 1 bis 20 ist, und n und o = 0 sind. Werden Alkohole der Formel **IVa** bei dem Verfahren verwendet, so erhält man stickstoffhaltige verbrückte Derivate von 6H-Dibenz[c,e][1,2]-oxaphosphorin-6-oxiden **IIa**, 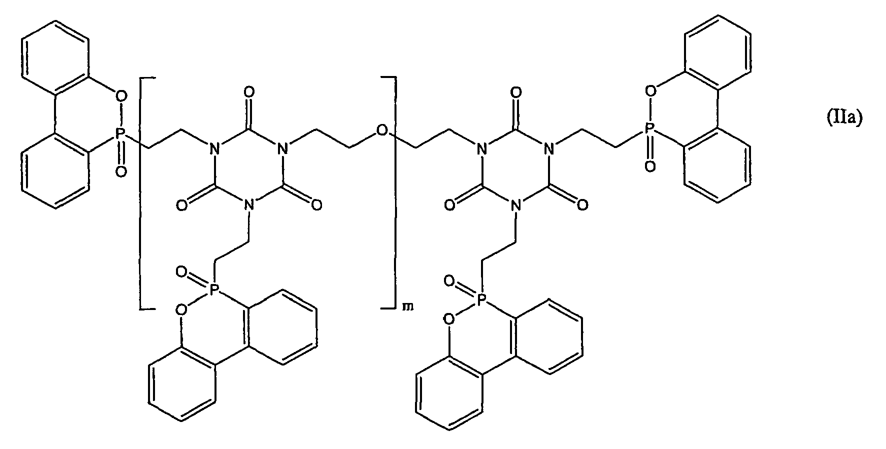 die sich von den Derivaten der Formel **II** ableiten, bei denen m eine ganze Zahl von 1 bis 20 ist, und n und o = 0 sind.

Bei einer bevorzugten Ausfütlrungsform wird der in Schritt (a) entstehende Alkohol, der Ethanol ist, wenn 6-Ethoxy-(6H)-dibenz[c,e][1,2]-oxaphosphorin verwendet wird, kontinuierlich aus dem Reaktionsgefäß entfernt. Das hat den Vorteil, daß durch das Entfernen des Alkohols das Reaktionsgleichgewicht auf die Seite des Zwischenproduktes verschoben wird.

Das in Stufe (a) erhaltene Zwischenprodukt läßt sich durch die folgenden Formeln **III'** und **IV'** bzw. **IVa'** (wenn der mehrwertige Alkohol der Formel **IVa** verwendet wird) beschreiben: 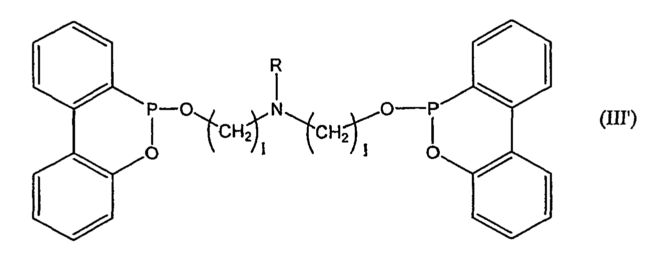 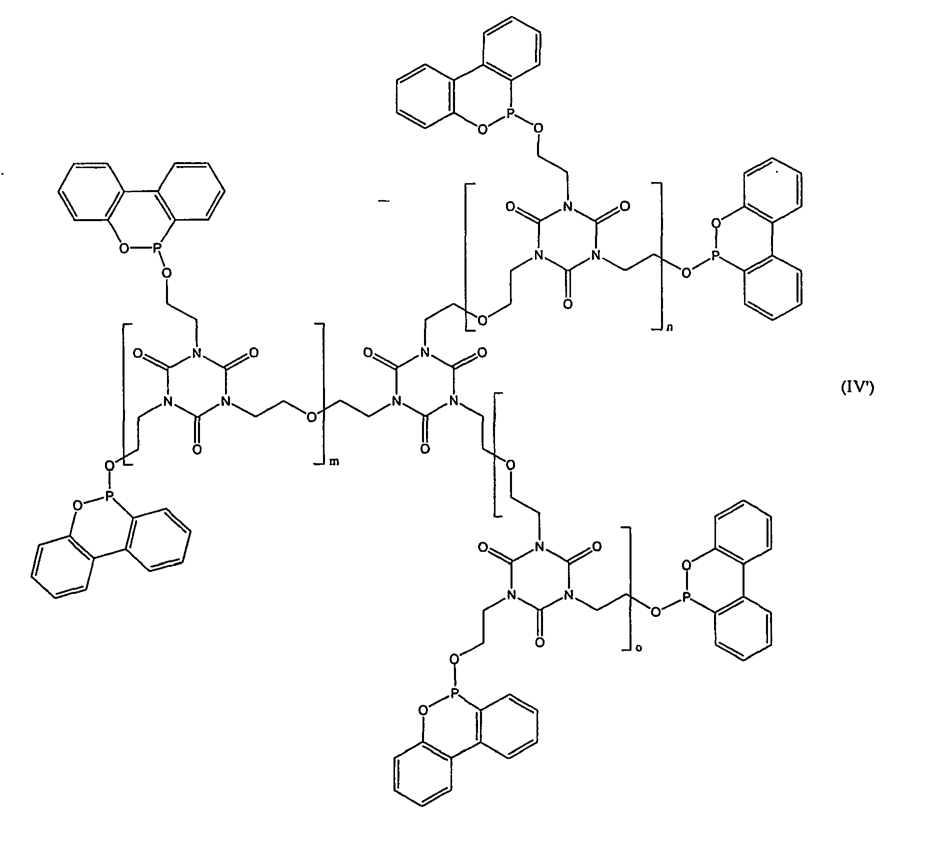 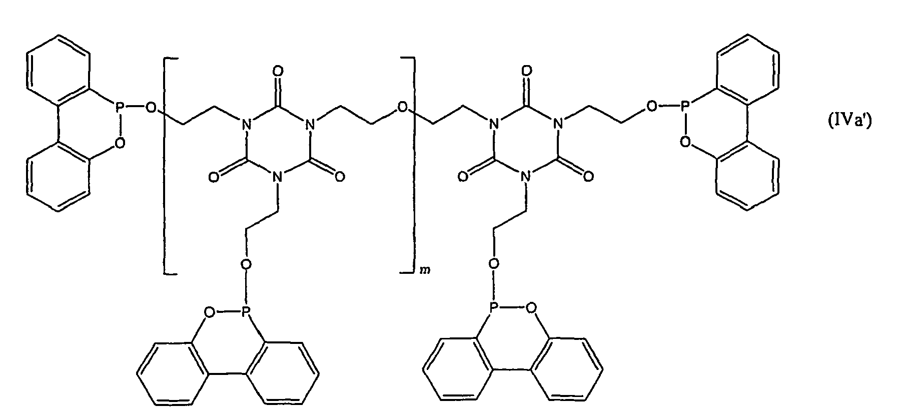 worin die Reste R und R' sowie l, m, n, o und p die oben angegebenen Bedeutungen haben.

Nach dem Ende der Reaktion in Schritt (a) wird in einer bevorzugten Ausführungsform der Erfindung das überschüssige 6-Alkoxy-(6H)-dibenz[c,e][1,2]-oxaphosphorin im Feinvakuum (0,01- 0,001 mbar) abdestilliert.

Anschließend werden die Zwischenprodukte, die sich im ersten Schritt gebildet haben, in einem zweiten Schritt (b) durch Zugabe katalytischer Mengen Alkylierungsmittel in die stickstoffhaltigen verbrückten Derivate von 6H-Dibenz[c,e][1,2]-oxaphosphorin-6-oxiden der Formel **I** oder **II** bzw. **IIa** mittels einer intramolekularen Michaelis-Arbusov-Reaktion übergeführt. Das halogenfreie Alkylierungsmittel wird in katalytischen Mengen von insbesondere 1 bis 10 mmol/mol zugesetzt, in dessen Gegenwart sich das bezogen auf Phophor dreibindige Zwischenprodukt in eine fünfbindige Phosphorverbindung umwandelt. Als Alkylierungsmittel können Schwefelsäureester sowie Sulfonsäureester verwendet werden. Bevorzugt sind Sulfonsäureester aromatischer Sulfonsäuren wie Alkyl-p-toluolsulfonsäureester, z.B. p-Toluolsulfonsäuremethylester. Reaktionsschritt (b) wird vorzugsweise bei erhöhten Temperaturen von über 170°C und unter Schutzgas, wie z.B. Argon oder Stickstoff, durchgeführt.

Das erfindungsgemäße Verfahren weist im Gegensatz zur klassischen Michaelis-Arbusov-Reaktion den Vorteil auf, daß man halogenfrei arbeiten kann, weil halogenfreie Alkylierungsmittel wie Schwefelsäureester oder Sulfonsäureester in katalytischen Mengen verwendet werden.

Nach der katalytischen Umsetzung in Schritt (b) wird das erhaltene Produkt abgetrennt und ggf. gereinigt und getrocknet.

Bevorzugte Verbindungen der Formel **I** sind solche, bei denen R eine Arylgruppe, insbesondere Phenyl, oder eine Arylsulfonylgruppe, insbesondere p-Toluolsulfonylgruppe, und 1= 2 ist.

Das erfindungsgemäße Verfahren hat insbesondere den Vorteil, daß es in Form einer Eintopfsynthese, d.h. in einem einzigen Reaktionsgefäß, mit Ausbeuten ohne aufwendige Reinigungsoperationen zwischen den Reaktionsstufen durchgeführt werden kann.

Die erfindungsgemäßen stickstoffhaltigen verbrückten Derivate von 6H-Dibenz[c,e][1,2]-oxaphosphorin-6-oxiden der Formel **I** oder **II** bzw. **IIa** eignen sich als Flammschutzmittel für Polymere, insbesondere für Polyester, Polyamid, Polycarbonat, Polystyrol, Polyethylen, Polypropylen, Phenol- oder Epoxidharz.

Die Erfindung betrifft ferner Oligomere der 1,3,5-Tris(2-hydroxyethyl)cyanursäure der Formel **IV** 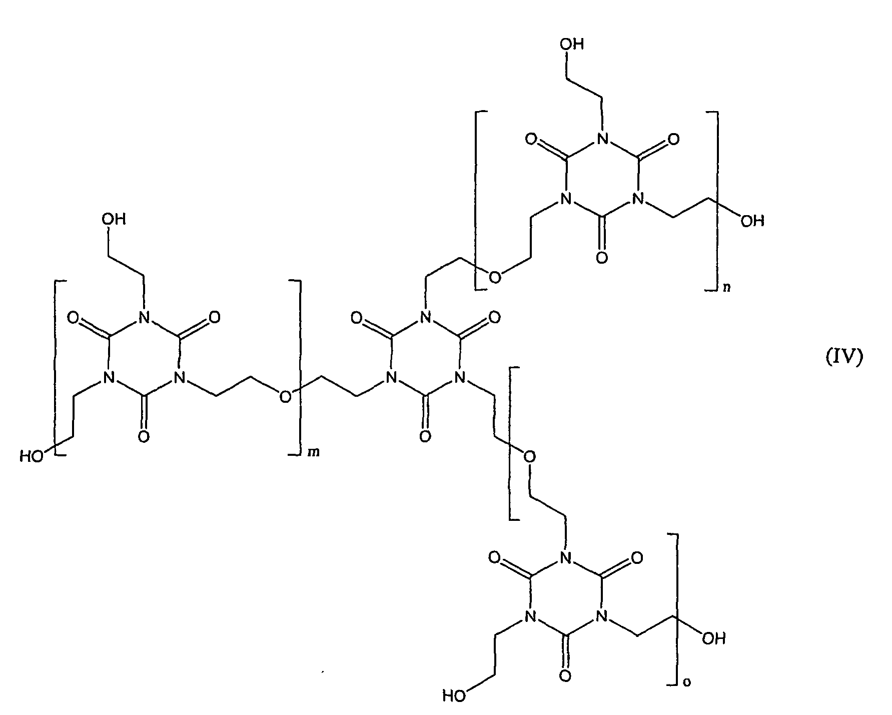 worin m eine ganze Zahl von 1 bis 20 und n und o ganze Zahlen von 0 bis 20 sind, und insbesondere Oligomere der Formel **IVa**, 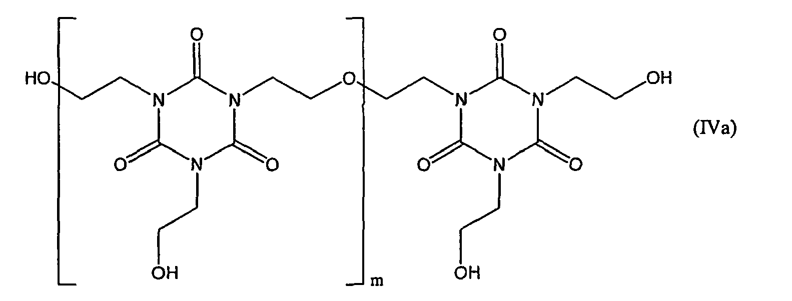 die sich von den Oligomeren der Formel **IV** ableiten, bei denen m eine ganze Zahl von 1 bis 20 ist, und n und o = 0 sind.

Die Oligomere der Formel **IV** bzw. **IVa** werden zur Herstellung der Derivate der Formel **II** bzw. **IIa** zusammen mit dem 6-Alkoxy-(6H)-dibenz[c,e][1,2]-oxaphosphorin in Schritt (a) umgesetzt.

Die Herstellung der Oligomere erfolgt ausgehend von 1,3,5-Tris(2-hydroxyethyl)cyanursäure, welche einer säurekatalysierten Polykondensation unterzogen wird.

Bevorzugte Ausführungsformen und Vorteile der vorliegenden Erfindung ergeben sich insbesondere aus den Beispielen.

### Beispiel 1

### Herstellung von IVa:

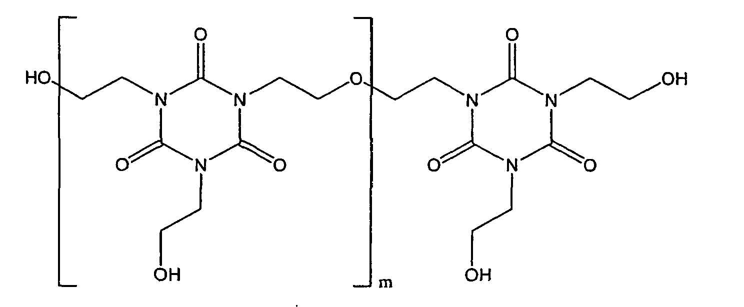

0,25 mol 1,3,5-Tris(2-hydroxyethyl)cyanursäure sowie 7 g Katalysator (am polymeren Träger gebundene p-Toluolsulfonsäure mit ca. 0,003 mol SO₃H-Gruppen pro Gramm) werden in einen Rundkolben, der mit einem Innenthermometer, einem Rückflußkühler und einem wirksamen Magnetrührer ausgestattet ist, auf 170°C erhitzt. Es wird ein leichter Stickstoffstrom über die Mischung geleitet, die nach dem Schmelzen der 1,3,5-Tris(2-hydroxyethyl)cyanursäure kräftig gerührt wird. In Abständen von 1 h wird das entstehende Wasser durch kurzzeitiges Anlegen von Vakuum entfernt. Die Reaktionsgeschwindigkeit ist zunächst gering, doch steigt sie allmählich an, erkennbar an einer vermehrten Abscheidung von Wassertröpfchen im Rückflußkühler. Nach einer Reaktionsdauer von 10 - 15 h bei 170°C steigt die Viskosität der Schmelze deutlich an. Die Umsetzung wird abgebrochen, wenn die Schmelze zwar dickflüssig aber noch rührbar ist (bei weiterer Fortführung der Kondensation würden vernetzte unlösliche Produkte erhalten). Vor dem Abkühlen wird nochmals Vakuum angelegt (ca. 1 mbar, wenige min). Bei ca. 80°C werden 250 ml abs. Dioxan zugegeben und die Mischung wird unter Stickstoff bis zum Sieden des Lösungsmittels erwärmt. Sie wird bis zur vollständigen Auflösung des Produktes am Sieden gehalten und nach dem Abkühlen durch eine 10 mm dicke Schicht Kieselgur filtriert, um den Katalysator vollständig zu entfernen. Zum klaren Filtrat werden 50 ml Toluol gegeben und anschließend werden die Lösungsmittel gemeinsam mit noch enthaltene Wasserresten bei ca. 50 mbar weitestgehend entfernt, wobei allmählich bis auf ca. 110°C erwärmt wird. Dann wird der noch warme Rückstand wieder in 130 ml abs. Dioxan gelöst und es werden wenige Tropfen Triethanolamin sowie 3 g Orthoameisensäureethylester zugesetzt, um eventuell noch enthaltene Säure- bzw. Wasserspuren zu entfernen. Durch Einengen auf ca. die Hälfte des Ausgangsvolumens bei 50 mbar wird eine konzentrierte Lösung der oligomerisierten 1,3,5-Tris(2-hydroxyethyl)cyanursäure erhalten.

### Beispiel 2

### Herstellung von Ia, (R = p-Toluolsulfonyl,1= 2):

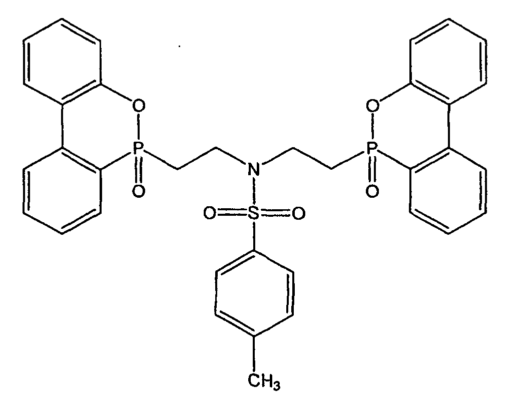

Eine Mischung von 20,75 g (0,080 mol) N,N-Bis(2-hydroxyethyl)-p-toluolsulfonamid und 44,96g (0,184 mol) 6-Ethoxy-[6H]-dibenz[c,e][1,2]-oxaphosphorin wird 18 h im Vakuum (ca. 10 mbar) bei 120°C gerührt. Anschließend wird das überschüssige 6-Ethoxy-[6H]-dibenz[c,e][1,2]-oxaphosphorin bei 0,001 mbar abdestilliert. Der Destillationsrückstand wird nach Zugabe von 1,3 g (0,007 mol) p-Toluolsulfonsäuremethylester unter Argon auf 180°C erhitzt. Diese Temperatur wird 24 h gehalten. Nach dem Abkühlen wird die Substanz in Methylenchlorid gelöst. Dann wird Diethylether zugesetzt, wobei das Rohprodukt ausfällt. Dieses wird abfiltriert und in warmem Methanol aufgelöst. Beim Abkühlen der methanolischen Lösung scheidet sich das Produkt feinkristallin aus. Es wird abfiltriert und bei ca. 80°C im Vakuum getrocknet. Die Ausbeute beträgt 14,1 g (30 %) **Ia**.

### Beispiel 3

### Herstellung von Ib, (R = Phenyl,1= 2):

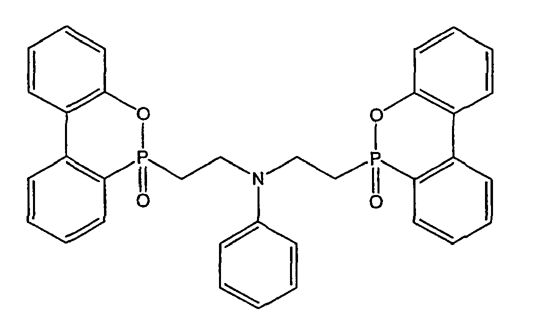

Eine Mischung von 10,98 g (0,045 mol) 6-Ethoxy-[6H]-dibenz[c,e][1,2]-oxaphosphorin und 2,85 g (0,0157 mol) N-Phenyldiethanolamin wird 18 h bei 120°C im Vakuum (ca. 10 mbar) gerührt. Anschließend wird das überschüssige 6-Ethoxy-[6H]-dibenz[c,e][1,2]-oxaphosphorin im Feinvakuum (0,001 mbar) abdestilliert. Nach Zugabe von 0,3 g (0,0016 mol) p-Toluolsulfonsäuremethylester wird der Destillationsrückstand unter Argon auf 190°C erhitzt und 19 h bei dieser Temperatur gehalten. Nach dem Abkühlen wird die Substanz in Methylenchlorid gelöst. Das Rohprodukt wird durch Zugabe von Diethylether ausgefällt. Es wird abgesaugt und in Methanol aufgelöst. Beim langsamen Abkühlen der methanolischen Lösung bis auf ca. -30°C wird reines **Ib** erhalten. Es wird abgesaugt und im Vakuum auf 70°C erwärmt (Ausbeute 4,98 g bzw. 55 %).

### Beispiel 4

### Herstellung von IIa:

Eine erfindungsgemäß hergestellte Lösung von **IVa** in Dioxan wird unter Stickstoff auf etwa 100°C erwärmt und es werden 139 g (0,57 mol) ebenfalls erwärmtes 6-Ethoxy-[6H]-dibenz[c,e][1,2]-oxaphosphorin unter kräftigem Rühren zugegeben. Dann wird das Dioxan bei ca. 50 mbar entfernt, wobei die Mischung allmählich auf 135°C erwärmt wird. Anschließend wird der Druck bis auf 1 mbar gesenkt, und es wird kräftig gerührt. Die Temperatur wird im Verlauf von 10 h allmählich von 135°C auf 160°C gesteigert. Dann wird noch 2 h bei 160°C und 1 mbar gerührt. Das im Vakuum abdestillierende Ethanol wird in einer Tiefkühlfalle aufgefangen. Die zunächst zweiphasige Mischung wird allmählich homogen und ihre Viskosität steigt deutlich an. Nach Beendigung der Reaktion wird das überschüssige Ethoxy-[6H]-dibenz[c,e][1,2]-oxaphosphorin im Feinvakuum (0,001 mbar) abdestilliert, wobei maximal auf 175°C erhitzt wird. Nach Zugabe von 0,74 g (0,004 mol) p-Toluolsulfonsäuremethylester wird der Destillationsrückstand unter Stickstoff 15 h auf 175°C teigartig zäh, es kann aber nach Erhitzen auf ca. 220°C aus dem Kolben entnommen werden. Es läßt sich nach Abkühlen auf Raumtemperatur leicht pulverisieren und hat eine zahlenmittlere Molmasse von Mₙ = 2000 - 5000 g/mol (abhängig vom Oligomerisierungsgrad des Ausgangsstoffes).

## Patentansprüche

1. Verfahren zur Herstellung von stickstoffhaltigen verbrückten Derivaten von 6H-Dibenz[c,e][1,2]-oxaphosphorin-6-oxiden der Formeln (I) und (II) 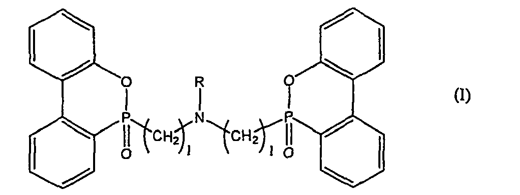 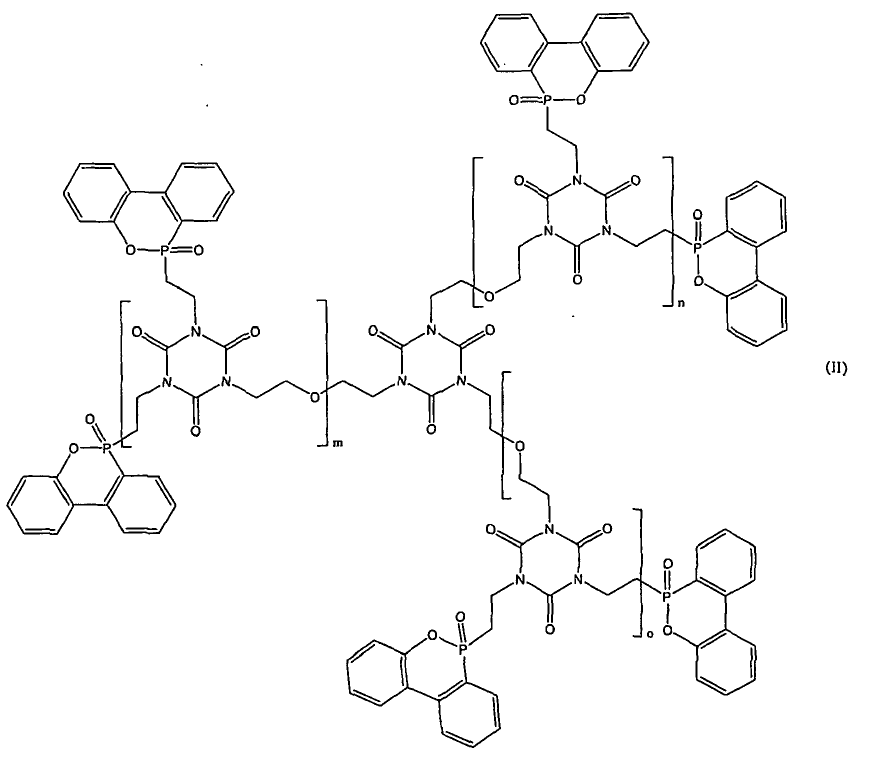 worin R einer der folgenden Reste: 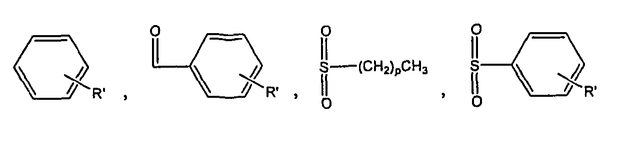 1 eine ganze Zahl von 2 bis 10, m und p ganze Zahlen von 1 bis 20, n und o ganze Zahlen von 0 bis 20 und R' = Wasserstoff oder Alkyl bedeuten, bei dem:
(a) ein 6-Alkoxy-[6H]-dibenz[c,e][1,2]-oxaphosphorin mit einem Bishydroxyalkylamin oder einem mehrwertigen Alkohol, der durch Polykondensation von 1,3,5-Tris(2-hydroxyethyl)cyanursäure gebildet wurde, unter Bildung eines Zwischenprodukts umgesetzt wird und
(b) das Zwischenprodukt aus Schritt (a) durch Zugabe katalytischer Mengen Alkylierungsmittel in ein stickstoffhaltiges verbrücktes 6H-Dibenz[c,e][1,2]-oxaphosphorin-6-oxid-Derivat der Formel (I) oder (II) übergeführt wird.

2. Verfahren nach Anspruch 1, **dadurch gekennzeichnet, daß** in Schritt (a) ein Bishydroxyalkylamin der Formel (III) oder ein mehrwertiger Alkohol der Formel (IV) verwendet wird, 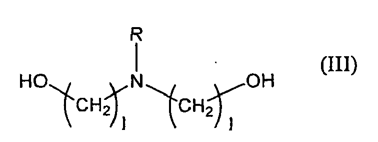 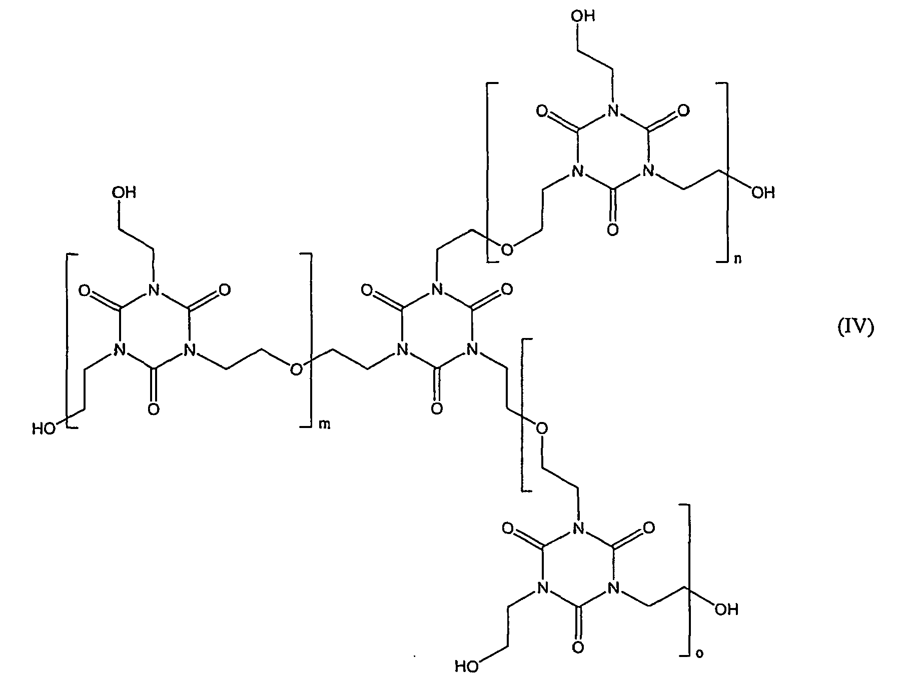 worin R einer der folgenden Reste: 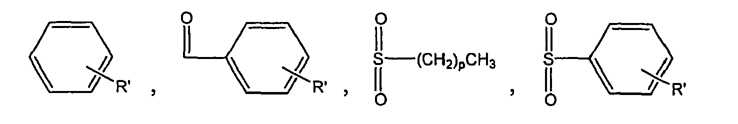 1 eine ganze Zahl von 2 bis 10, m und p ganze Zahlen von 1 bis 20, n und o ganze Zahlen von 0 bis 20 und R' = Wasserstoff oder Alkyl sind.

3. Verfahren nach Anspruch 1 oder 2, **dadurch gekennzeichnet, daß** stickstoffhaltige verbrückte Derivaten von 6H-Dibenz[c,e][1,2]-oxaphosphorin-6-oxiden der Formeln (IIa) hergestellt werden, 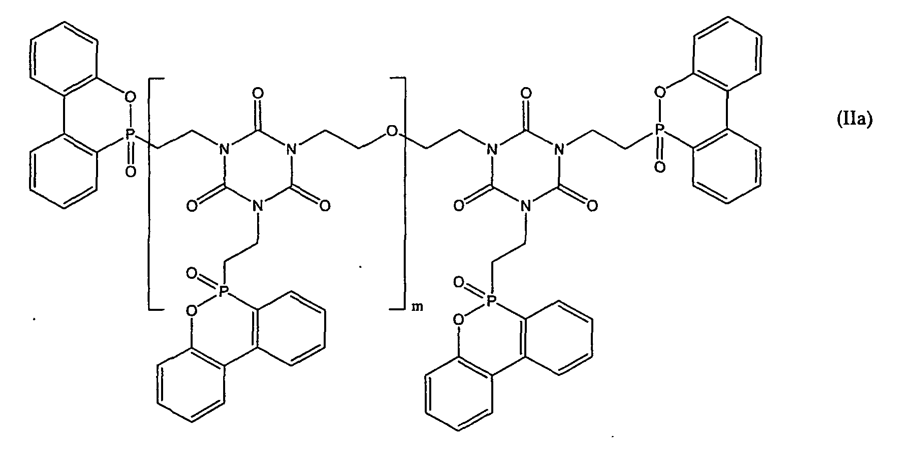 die sich von stickstoffhaltigen verbrückten Derivaten von 6H-Dibenz[c,e][1,2]-oxaphosphorin-6-oxiden der Formeln (II) ableiten, bei denen m eine ganze Zahl von 1 bis 20 ist, und n und o = 0 sind.

4. Verfahren nach einem der Ansprüche 1 bis 3, **dadurch gekennzeichnet, daß** in Schritt (a) ein mehrwertiger Alkohol der Formel (IVa) verwendet wird, 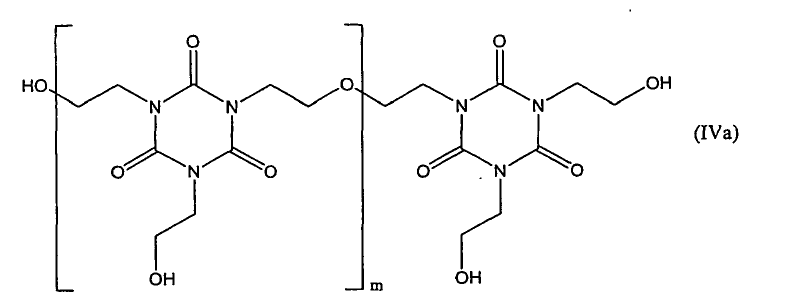 der sich aus dem mehrwertigen Alkohol der Formel (IV) ableitet, bei dem m eine ganze Zahl von 1 bis 20 ist, und n und o = 0 sind.

5. Verfahren nach Anspruch 1 oder 2, **dadurch gekennzeichnet, daß** in Schritt (a) ein Bishydroxyalkylamin verwendet wird, bei dem R eine Phenyl- oder p-Toluolsulfonylgruppe ist.

6. Verfahren nach Anspruch 2 oder 5, **dadurch gekennzeichnet, daß** in Schritt (a) Bis(hydroxyethyl)phenylamin oder Bis(hydroxyethyl)p-toluolsulfonylamin als Bishydroxyalkylamin verwendet wird.

7. Verfahren nach einem der Ansprüche 1 bis 6, **dadurch gekennzeichnet, daß** in Schritt (a) 6-Ethoxy-[6H]-dibenz[c,e][1,2]-oxaphosphorin eingesetzt wird.

8. Verfahren nach einem der Ansprüche 1 bis 7, **dadurch gekennzeichnet, daß** in Schritt (b) ein Alkylierungsmittel verwendet wird, das aus der Gruppe ausgewählt ist, die aus Schwefelsäureestern und Sulfonsäureestern besteht.

9. Verfahren nach Anspruch 8, **dadurch gekennzeichnet, daß** das Alkylierungsmittel, welches in Schritt (b) verwendet wird, p-Toluolsulfonsäuremethylester ist.

10. Verfahren nach einem der Ansprüche 1 bis 9, **dadurch gekennzeichnet, daß** der in Schritt (a) gebildete Alkohol entfernt wird.

11. Verfahren nach einem der Ansprüche 1 bis 10, **dadurch gekennzeichnet, daß** die Schritte (a) und (b) in einem einzigen Reaktionsgefäß durchgeführt werden.

12. Verfahren nach einem der Ansprüche 1 bis 11, **dadurch gekennzeichnet, daß** in einem mit Rückflußkühler und Rührer ausgestatteten Reaktionsgefäß unter ständigem Rühren nacheinander folgende Schritte durchgeführt werden:
(a) 6-Ethoxy-[6H]-dibenz[c,e][1,2]-oxaphosphorin und das Bishydroxyalkylamin oder der mehrwertige Alkohol werden bei Raumtemperatur in das Reaktionsgefäß gegeben und miteinander vermischt;
(b) das erhaltene Gemisch wird erhitzt unter gleichzeitigem Abdestillieren von entstandenem Ethanol;
(c) zu dem Destillationsrückstand wird der p-Toluolsulfonsäuremethylester gegeben und das erhaltene Gemisch wird erhitzt,
wonach die Verbindung der Formel (I) oder (II) abgetrennt und gegebenenfalls gereinigt und getrocknet wird.

13. Verfahren nach Anspruch 12, **dadurch gekennzeichnet, daß** im Anschluß an Schritt (b) überschüssiges 6-Ethoxy-[6H]-dibenz[c,e][1,2]-oxaphosphorin im Feinvakuum (0,01 - 0,001 mbar) abdestilliert wird.

14. Stickstoffhaltige verbrückte Derivate von 6H-Dibenz[c,e][1,2]-oxaphosphorin-6-oxiden der Formeln (I) und (II) 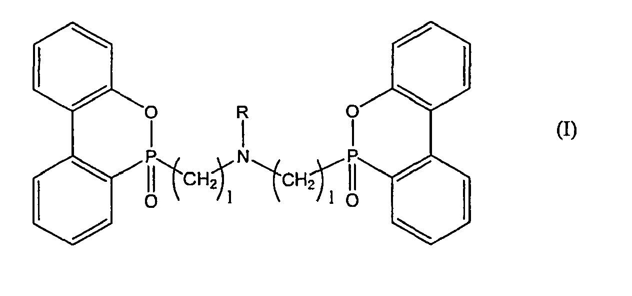 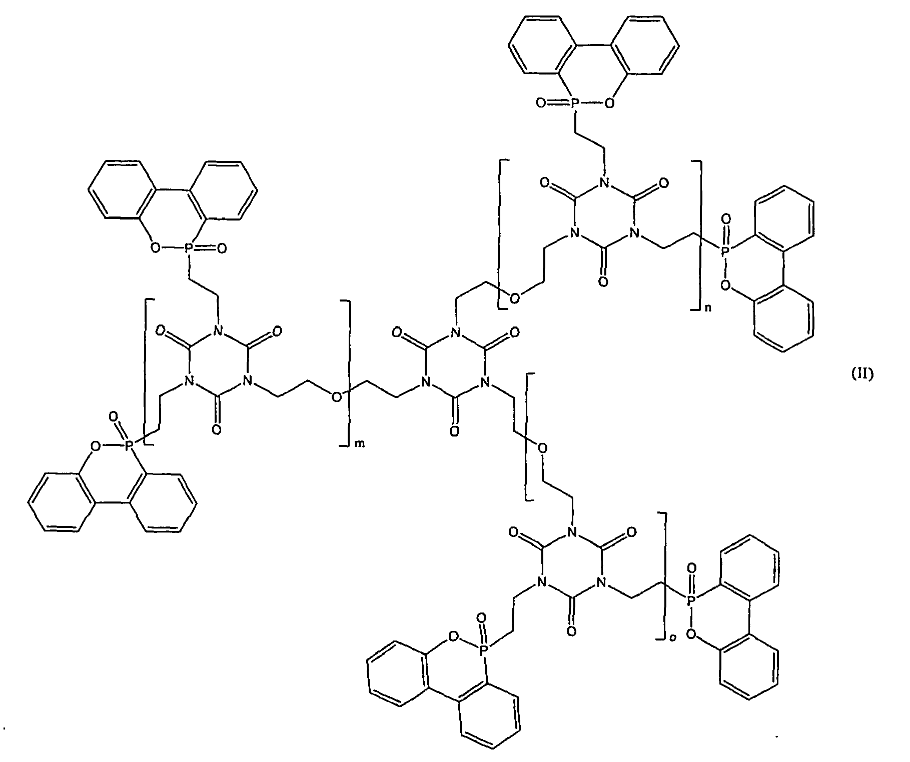 worin R einer der folgenden Reste: 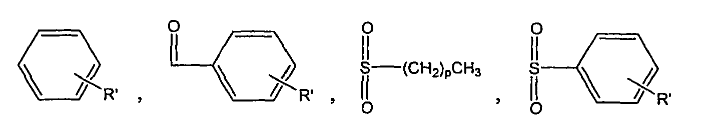 1 eine ganze Zahl von 2 bis 10, m und p ganze Zahlen von 1 bis 20, n und o ganze Zahlen von 0 bis 20 und R' = Wasserstoff oder Alkyl sind.

15. Stickstoffhaltige verbrückte Derivate von 6H-Dibenz[c,e][1,2]-oxaphosphorin-6-oxiden nach Anspruch 14, **dadurch gekennzeichnet, daß** R eine Phenyl- oder p-Toluolsulfonylgruppe ist.

16. Stickstoffhaltige verbrückte Derivate von 6H-Dibenz[c,e][1,2]-oxaphosphorin-6-oxiden nach Anspruch 14 oder 15, **dadurch gekennzeichnet, daß** R eine Phenyl- oder p-Toluolsulfonylgruppe und 1= 2 ist.

17. Stickstoffhaltige verbrückte Derivate von 6H-Dibenz[c,e][1,2]-oxaphosphorin-6-oxiden nach Anspruch 14, **dadurch gekennzeichnet, daß** sie eine Struktur der Formel (IIa) aufweisen, 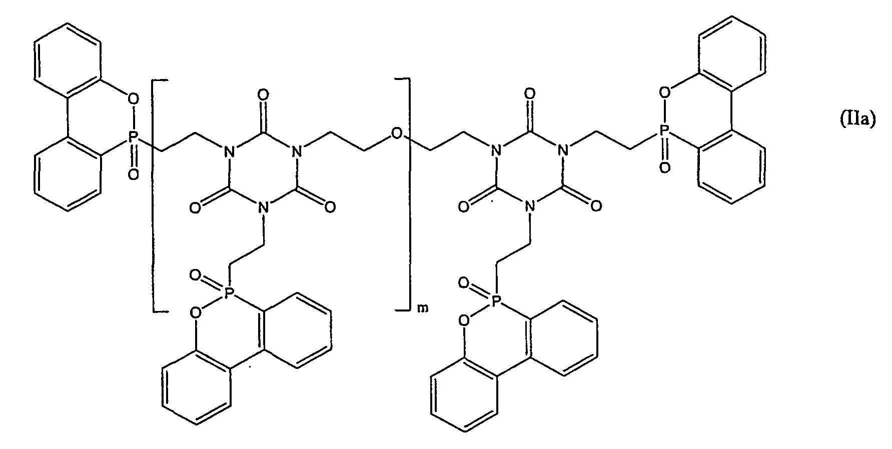 die sich von Formel (II) ableitet, bei der m eine ganze Zahl von 1 bis 20 ist, und n und o = 0 sind.

18. Verwendung von 6H-Dibenz[c,e][1,2]-oxaphosphorin-6-oxid-Derivaten, die nach einem Verfahren gemäß den Ansprüchen 1 bis 13 hergestellt wurden, als Flammschutzmittel für Polymere und daraus hergestellte Erzeugnisse.

19. Verwendung nach Anspruch 18, als Flammschutzmittel für Polyester, Polyamide Polycarbonate, Polystyrole, Polyethylene, Polypropylene, Phenol- oder Epoxidharze.

20. Oligomere der 1,3,5-Tris(2-hydroxyethyl)cyanursäure der Formel (IV) 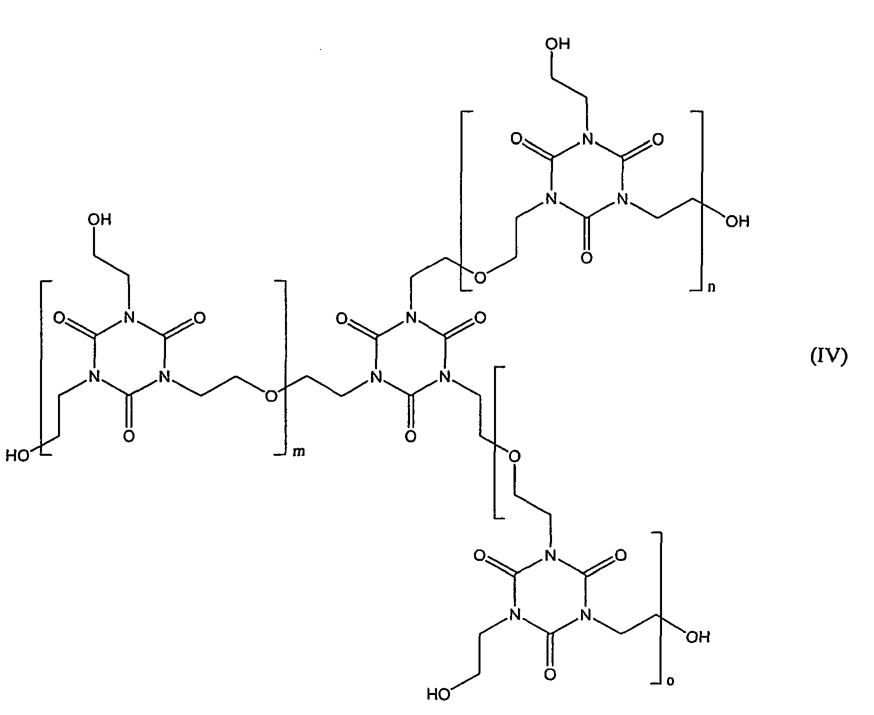 worin m eine ganze Zahl von 1 bis 20 und n und o ganze Zahlen von 0 bis 20 sind.

21. Oligomere nach Anspruch 20 mit der Formel (IVa), 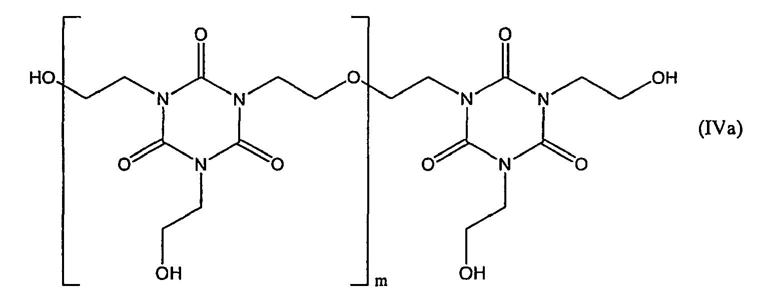 deren Struktur sich von Formel (IV) ableitet, bei der m eine ganze Zahl von 1 bis 20 ist, und n und o = 0 sind.

## Claims

1. A process for preparation of nitrous bridged derivatives of 6H-dibenz[c,e][1,2]-oxaphosphorine-6-oxides of Formulae (I) and (II) 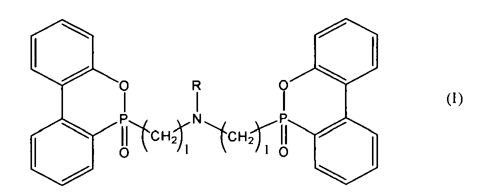 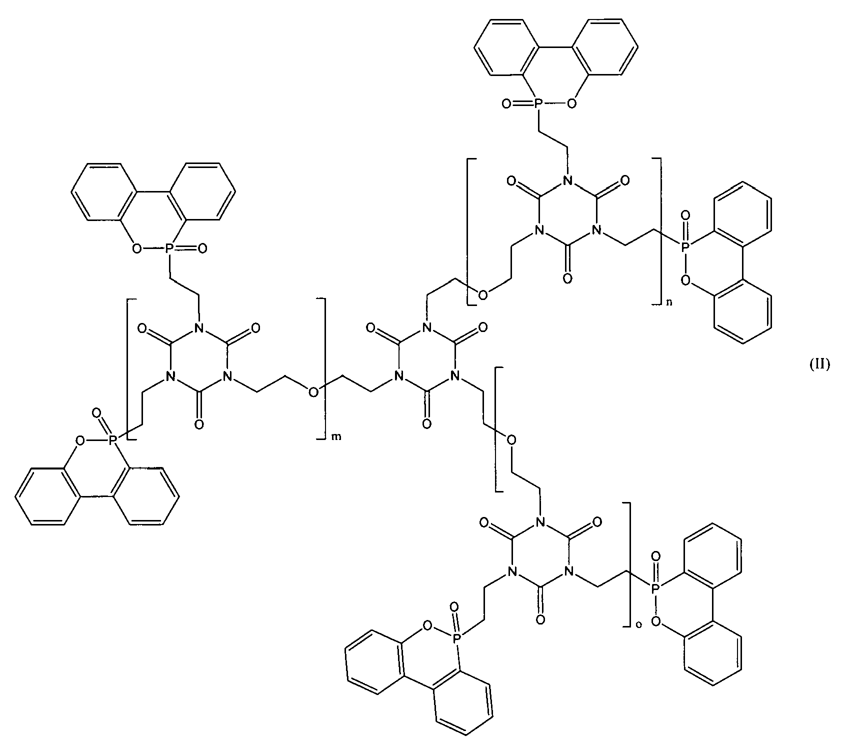 wherein R is one of the following radicals: 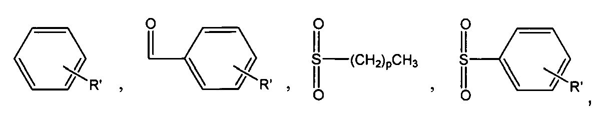 I is an integer of from 2 to 10, m and p are integers of from 1 to 20, n and o are integers of from 0 to 20, and R' is hydrogen or alkyl, wherein:
(a) a 6-alkoxy-[6H]-dibenz[c,e][1,2]-oxaphosphorine is reacted with a bishydroxyalkyl amine or a polyvalent alcohol formed by polycondensation of 1,3,5-tris(2-hydroxyethyl)cyanuric acid, to form an intermediate product, and
(b) the intermediate product obtained in step (a) is converted by adding a catalytic amount of an alkylating agent to a nitrous bridged 6H-dibenz[c,e][1,2]-oxaphosphorine-6-oxide derivative of the Formula (I) or (II).

2. The process according to claim 1, **characterized in that** in step (a) there is used a bishydroxyalkyl amine of the Formula (III) or a polyvalent alcohol of the Formula (IV) 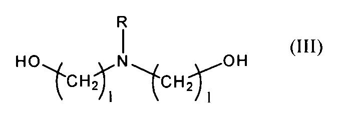 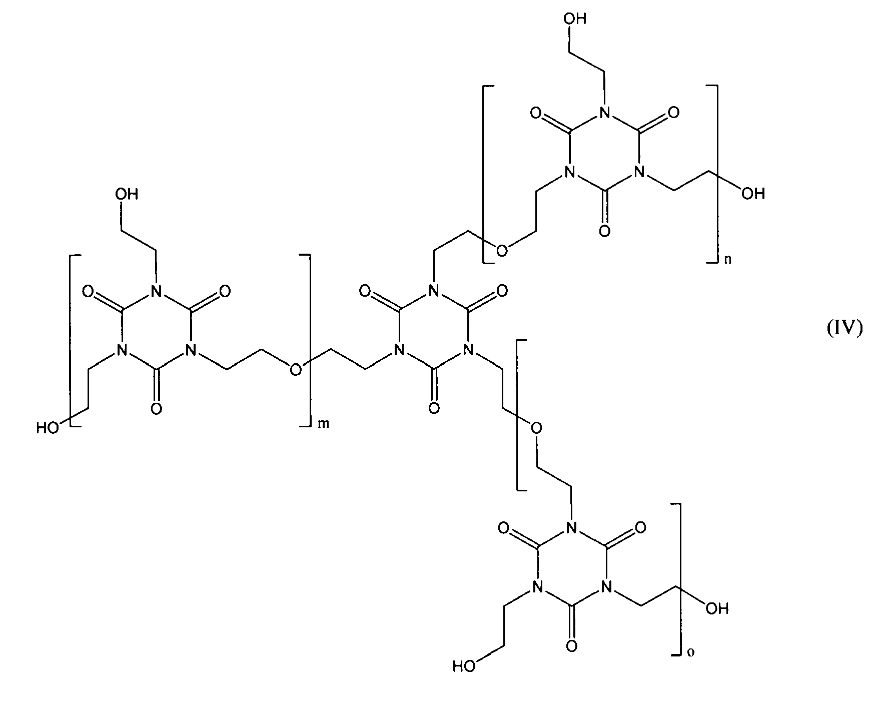 wherein R is one of the following radicals: 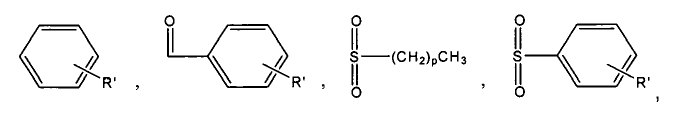 I is an integer of from 2 to 10, m and p are integers of from 1 to 20, n and o are integers of from 0 to 20, and R' is hydrogen or alkyl.

3. The process according to claim 1 or 2, **characterized in that** there are produced nitrous bridged derivatives of 6H-dibenz[c,e][1,2]-oxaphosphorine-6-oxides of the Formula (IIa) 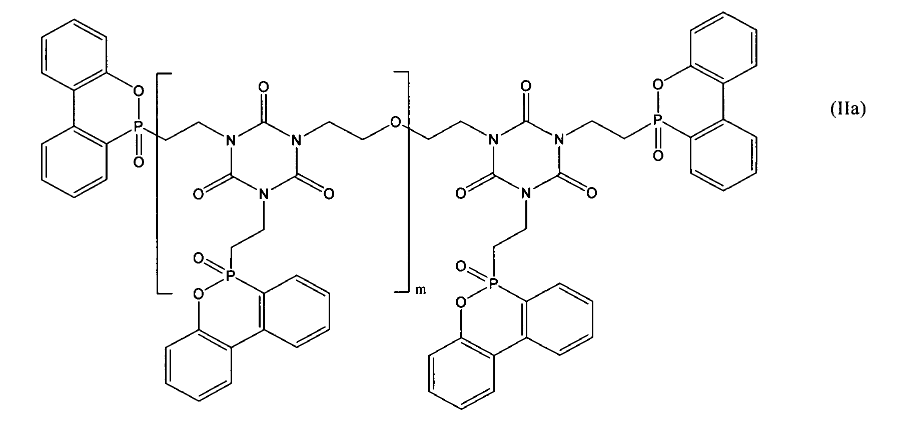 which derive from nitrous bridged derivatives of 6H-dibenz[c,e][1,2]-oxaphosphorine-6-oxides of the Formula (II) in which m is an integer of from 1 to 20, and n and o are 0.

4. The process according to any of claims 1 to 3, **characterized in that** in step (a) there is used a polyvalent alcohol of the Formula (IVa) 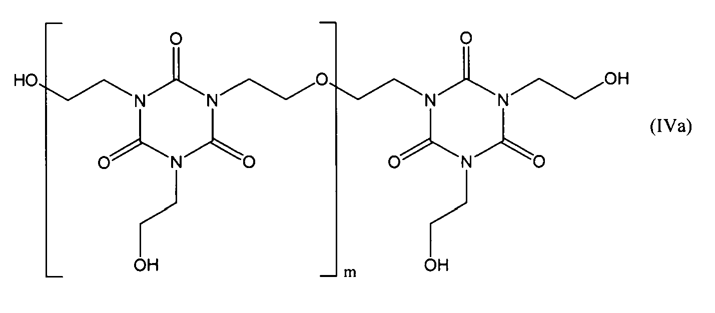 derived from the polyvalent alcohol of the Formula (IV) in which m is an integer of from 1 to 20, and n and o are 0.

5. The process according to claim 1 or 2, **characterized in that** a bishydroxyalkyl amine where R is a phenyl or p-toluenesulfonyl group is used in step (a).

6. The process according to claim 2 or 5, **characterized in that** bis(hydroxyethyl) phenyl amine or bis(hydroxyethyl)p-toluenesulfonylamine is used as bishydroxyalkyl amine in step (a).

7. The process according to any of claims 1 to 6, **characterized in that** 6-ethoxy-[6H]-dibenz[c,e][1,2]-oxaphosphorine is used in step (a).

8. The process according to any of claims 1 to 7, **characterized in that** in step (b) an alkylating agent is used which is selected from the group consisting of sulfuric acid esters and sulfonic acid esters.

9. The process according to claim 8, **characterized in that** the alkylating agent used in step (b) is p-toluene sulfonic acid methyl ester.

10. The process according to any of claims 1 to 9, **characterized in that** the alcohol formed in step (a) is removed.

11. The process according to any of claims 1 to 10, **characterized in that** steps (a) and (b) are carried out in a single reaction vessel.

12. The process according to any of claims 1 to 11, **characterized in that** in a reaction vessel equipped with a reflux cooler and a stirrer the following steps are carried out in succession while constantly stirring:
(a) 6-ethoxy-[6H]-dibenz[c,e][1,2]-oxaphosphorine and the bishydroxyalkyl amine or the polyvalent alcohol are put at room temperature in the reaction vessel and mixed with each other;
(b) the mixture obtained is heated while simultaneously distilling off of ethanol generated;
(c) the p-toluene sulfonic acid methyl ester is added to the distillation residue, and the mixture obtained is heated,
after which the compound of Formula (I) or (II) is separated and, optionally, cleaned and dried.

13. The process according to claim 12, **characterized in that** subsequent to step (b), excess 6-ethoxy-[6H]-dibenz[c,e][1,2]-oxaphosphorine is distilled off under high-medium vacuum (0.01 - 0.001 mbar).

14. Nitrous bridged derivatives of 6H-dibenz[c,e][1,2]-oxaphosphorine-6-oxides of the Formulae (I) and (II) 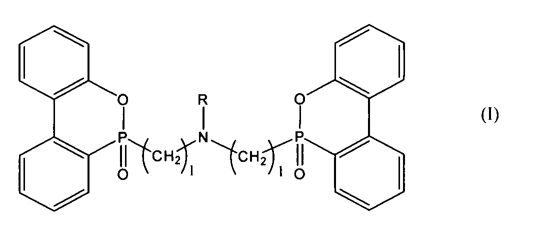 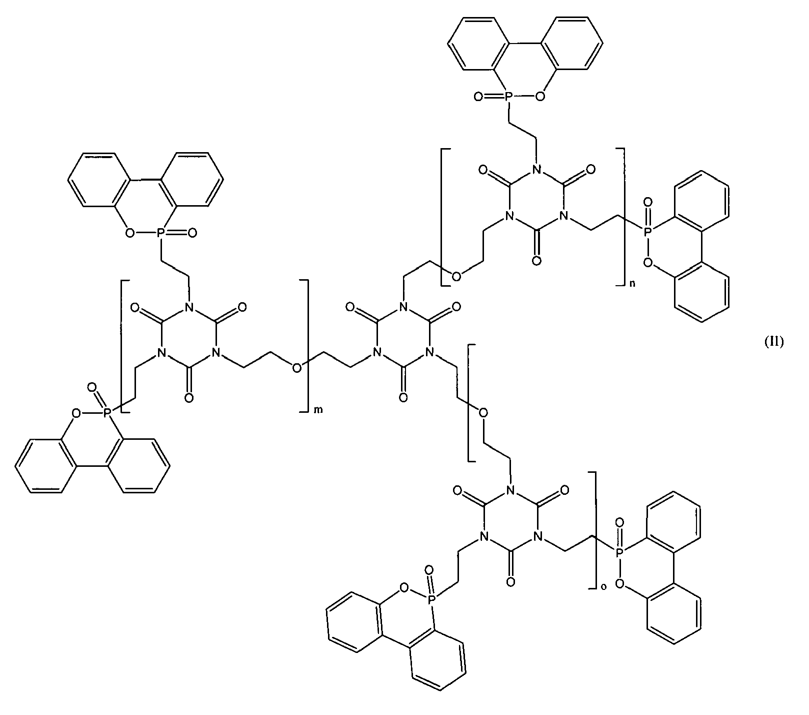 wherein R is one of the following radicals: 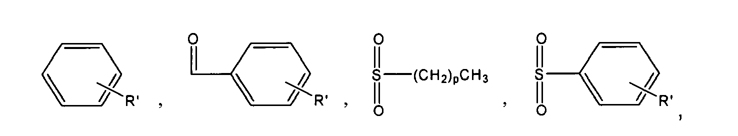 I is an integer of from 2 to 10, m and p are integers of from 1 to 20, n and o are integers of from 0 to 20, and R' is hydrogen or alkyl.

15. The nitrous bridged derivatives of 6H-dibenz[c,e][1,2]-oxaphosphorine-6-oxides according to claim 14, **characterized in that** R is a phenyl or p-toluenesulfonyl group.

16. The nitrous bridged derivatives of 6H-dibenz[c,e][1,2]-oxaphosphorine-6-oxides according to claim 14 or 15, **characterized in that** R is a phenyl or p-toluenesulfonyl group and I equals 2.

17. The nitrous bridged derivatives of 6H-dibenz[c,e][1,2]-oxaphosphorine-6-oxides according to claim 14, **characterized by** having a structure of the Formula (IIa) 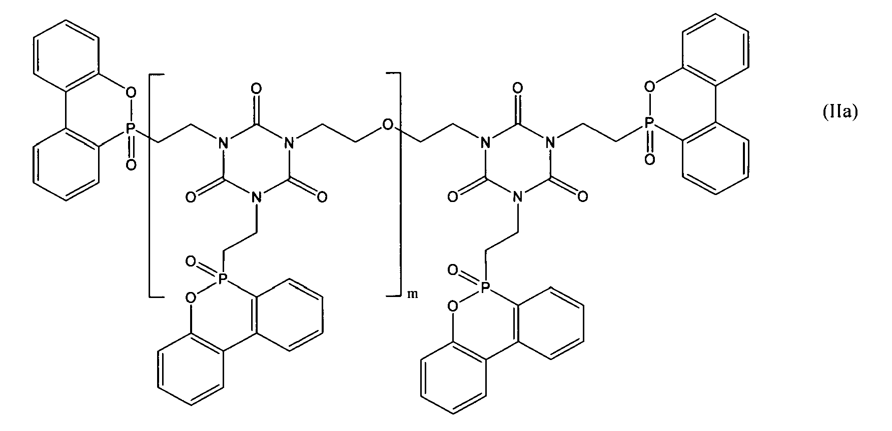 which is derived from Formula (II), in which m is an integer of from 1 to 20, and n and o are 0.

18. Use of 6H-dibenz[c,e][1,2]-oxaphosphorine-6-oxide derivatives prepared by a process according to claims 1 to 13 as flameproofing agents for polymers and products prepared therefrom.

19. The use according to claim 18 as flameproofing agents for polyesters, polyamides, polycarbonates, polystyrenes, polyethylenes, polypropylenes, phenolic and epoxy resins.

20. Oligomers of the 1,3,5-tris(2-hydroxyethyl)cyanuric acid of the Formula (IV) 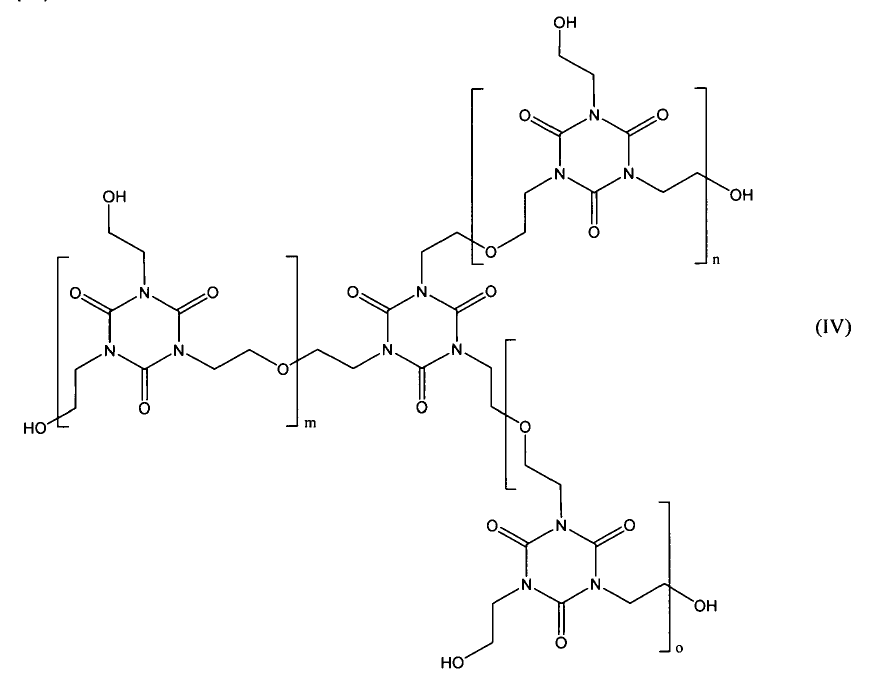 wherein m is an integer of from 1 to 20, and n and o are integers of from 0 to 20.

21. The oligomers according to claim 20, having the Formula (IVa) 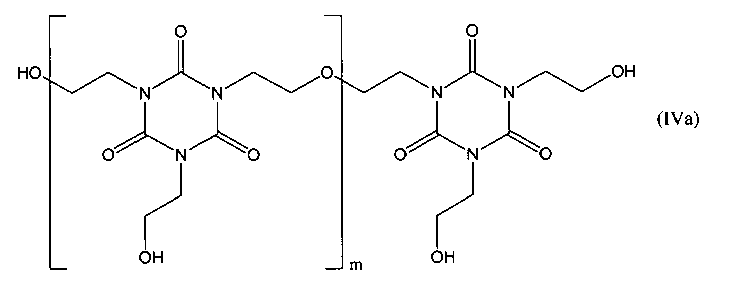 the structure of which derives from Formula (IV), in which m is an integer of from 1 to 20, and n and o are 0.

## Revendications

1. Procédé de fabrication de dérivés azotés à pont de 6H-dibenzo[c,e][1,2]-oxaphosphorine-6-oxides des formules (I) et (II) 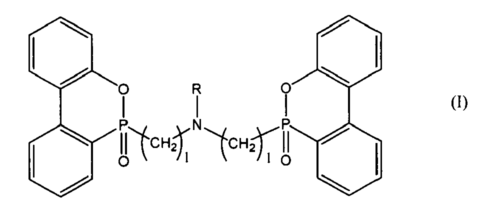 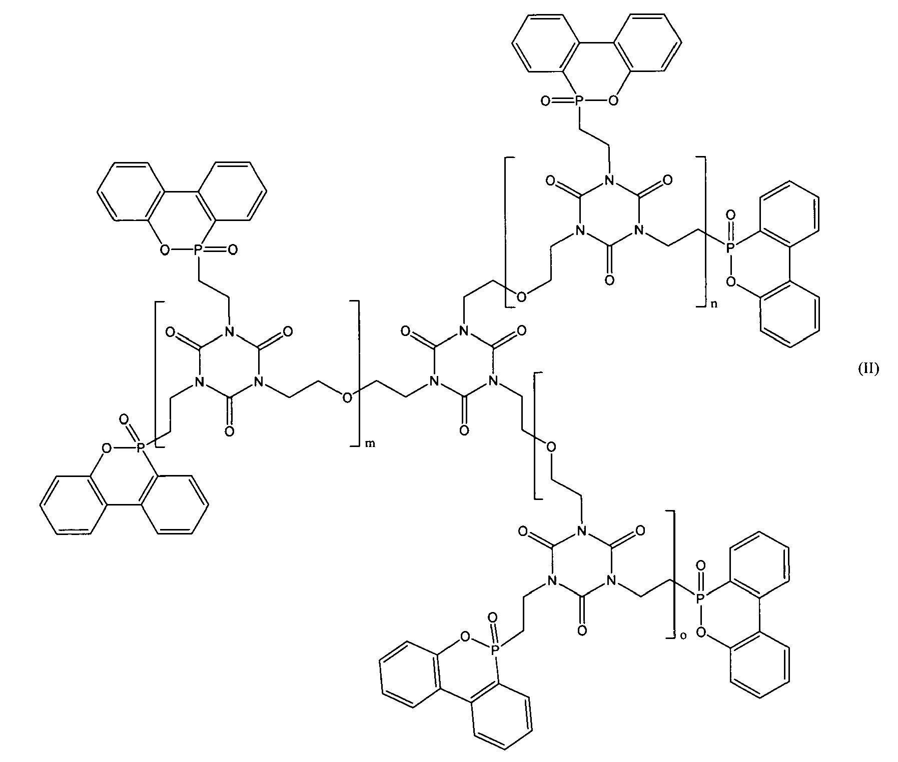 dans lesquelles R est un des radicaux suivants : 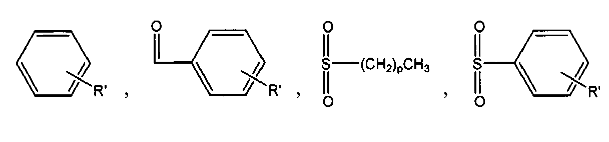 I désigne un nombre entier compris entre 2 et 10, m et p désignent des nombres entiers compris entre 1 et 20, n et o désignent des nombres entiers compris entre 0 et 20 et R' = hydrogène ou alkyle, dans lequel :
(a) on fait réagir une 6-Alkoxy-[6H]-dibenzo[c,e][1,2]-oxaphosphorine avec une bishydroxyalkylamine ou un alcool polyvalent qui a été formé par polycondensation d'acide 1,3,5-tris(2-hydroxyéthyle) cyanurique, en formant un produit intermédiaire , et
(b) on convertit le produit intermédiaire de l'étape (a) par ajout de quantité catalytique d'agent d'alkylation en un dérivé azoté à pont de 6H-dibenzo[c,e][1,2]-oxaphosphorine-6-oxide de la formule (I) ou (II).

2. Procédé selon la revendication 1, **caractérisé en ce qu'**à l'étape (a), on utilise une bishydroxyalkylamine de la formule (III) ou un alcool polyvalent de la formule (IV), 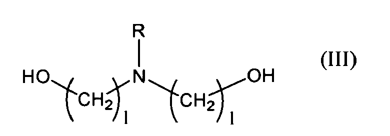 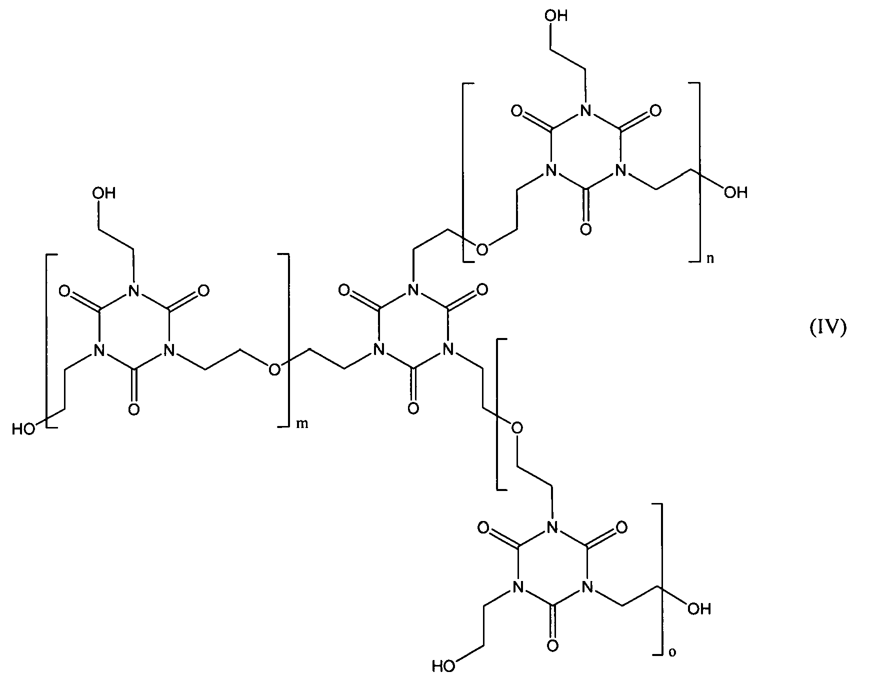 dans laquelle R est un des radicaux suivants : 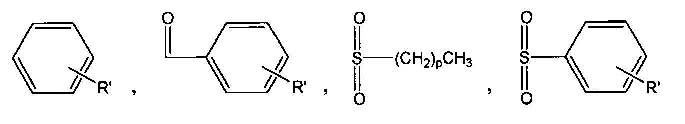 I est un nombre entier entre 2 et 10, m et p sont des nombres entiers entre 1 et 20, n et o sont des nombres entiers entre 0 et 20 et R' = hydrogène ou alkyle.

3. Procédé selon la revendication 1 ou 2, **caractérisé en ce qu'**on produit des dérivés azotés à pont de 6H-dibenzo[c,e][1,2]-oxa-phosphorine-6-oxides des formules (IIa), 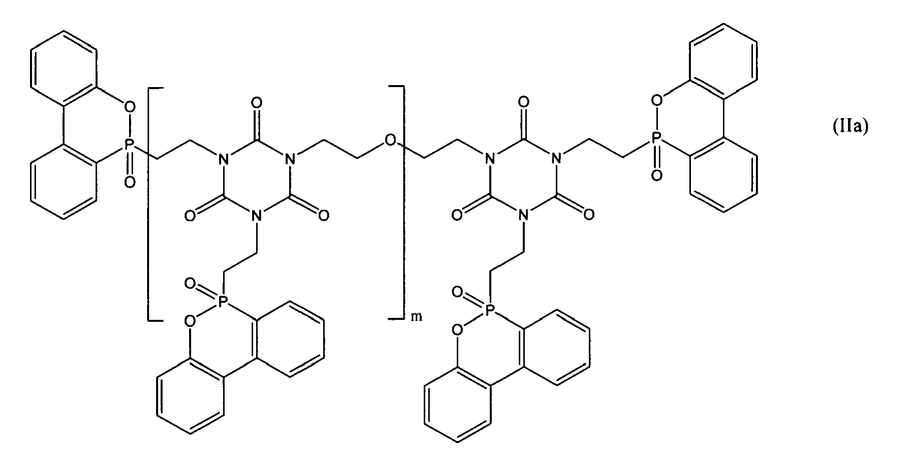 qui dérivent de dérivés azotés à pont de 6H-dibenzo[c,e][1,2]-oxaphosphorine-6-oxides des formules (II), dans lesquelles m est un nombre entier entre 1 et 20, et n et o = 0.

4. Procédé selon l'une des revendications 1 à 3, **caractérisé en ce qu'**on utilise à l'étape (a) un alcool polyvalent de la formule (IVa), 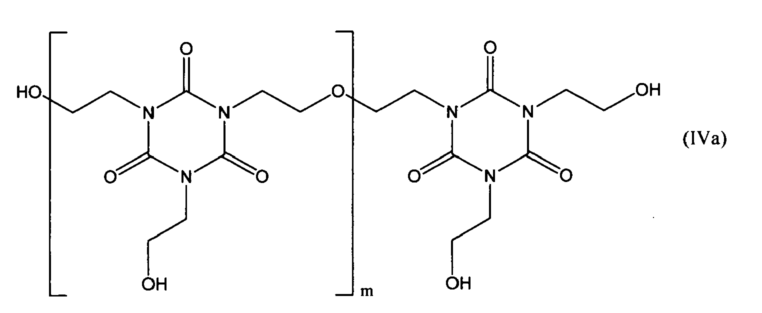 qui est dérivé de l'alcool polyvalent de la formule (IV) dans laquelle m est un nombre entier entre 1 et 20, et n et o = 0.

5. Procédé selon la revendication 1 ou 2, **caractérisé en ce qu'** à l'étape (a), on utilise une bishydroxyalkylamine dans laquelle R est un groupe de phényle ou de para-toluènesulfonyle.

6. Procédé selon la revendication 2 ou 5, **caractérisé en ce qu'** à l'étape (a), on utilise de la bis(hydroxyéthyle)phénylamine ou de la bis(hydroxyéthyle)paratoluènesulfonylamine à titre de bishydroxyalkylamine.

7. Procédé selon une des revendications 1 à 6, **caractérisé en ce qu'**à l'étape (a), on met en oeuvre de la 6-éthoxy-[6H]-dibenzo[c,e][1,2]-oxaphosphorine.

8. Procédé selon l'une des revendications 1 à 7, **caractérisé en ce qu'**à l'étape (b), on utilise un agent d'alkylation qui est choisi parmi le groupe constitué par des esters d'acide sulfurique et des esters d'acide sulfonique.

9. Procédé selon la revendication 8, **caractérisé en ce que** l'agent d'alkylation qui est utilisé à l'étape (b) est de l'ester méthylique de l'acide paratoluène sulfonique.

10. Procédé selon l'une des revendications 1 à 9, **caractérisé en ce qu'**on extrait l'alcool formé à l'étape (a).

11. Procédé selon l'une des revendications 1 à 10, **caractérisé en ce que** les étapes (a) et (b) sont exécutées dans un seul récipient de réaction.

12. Procédé selon l'une des revendications 1 à 11, **caractérisé en ce que** les étapes suivantes sont exécutées les unes après les autres dans un récipient de réaction équipé d'un condenseur à reflux et d'un agitateur, sans cesser de remuer :
(a) on place à température ambiante dans un récipient de réaction et on mélange l'une à l'autre de la 6-éthoxy-[6H]-dibenzo[c,e][1,2]-oxaphosphorine et la bishydroxyalkylamine ou l'alcool polyvalent;
(b) on chauffe le mélange obtenu en éliminant simultanément l'éthanol produit par distillation;
(c) on ajoute au résidu de distillation l'ester méthylique de l'acide paratoluène sulfonique et on chauffe le mélange obtenu,
à la suite de quoi le composé de la formule (I) ou (II) est séparé et éventuellement purifié et séché.

13. Procédé selon la revendication 12, **caractérisé en ce qu'**après l'étape (b), de la 6-éthoxy-[6H]-dibenzo[c,e][1,2]-oxaphosphorine excédentaire est éliminée par distillation en vide poussé (0,01 - 0,001 mbar).

14. Dérivés azotés à pont de 6H-dibenzo[c,e][1,2]-oxaphosphorine-6-oxides des formules (I) et (II) 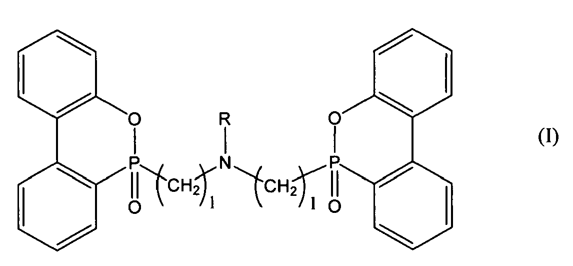 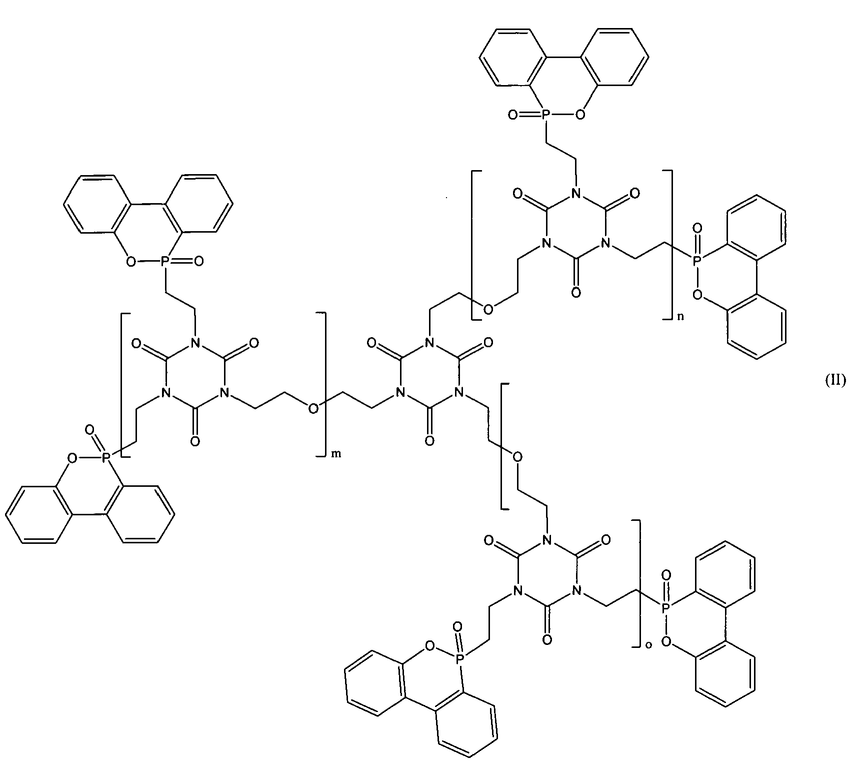 dans lesquelles R est un des radicaux suivants : 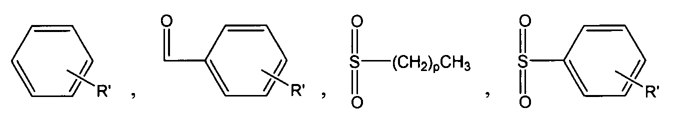 I est un nombre entier entre 2 et 10, m et p sont des nombres entiers entre 1 à 20, n et o sont des nombres entiers entre 0 et 20 et R' = hydrogène ou alkyle.

15. Dérivés azotés à pont de 6H-dibenzo[c,e][1,2]-oxaphosphorine-6-oxides selon la revendication 14, **caractérisés en ce que** R est un groupe de phényle ou de para-toluènesulfonyle.

16. Dérivés azotés à pont de 6H-dibenzo[c,e][1,2]-oxaphosphorine-6-oxides selon la revendication 14 ou 15, **caractérisé en ce que** R est un groupe de phényle ou de para-toluènesulfonyle et I = 2.

17. Dérivés azotés à pont de 6H-dibenzo[c,e][1,2]-oxaphosphorine-6-oxides selon la revendication 14, **caractérisés en ce qu'**ils présentent une structure de la formule (IIa), 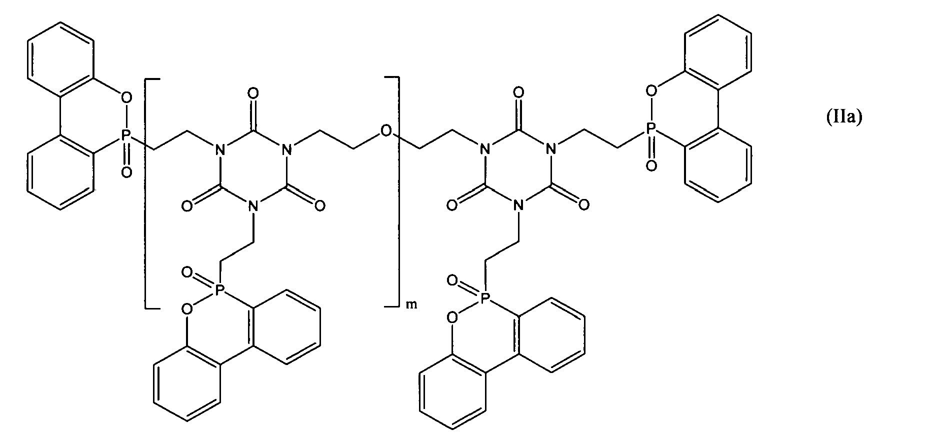 qui dérive de la formule (II), dans laquelle m est un nombre entier entre 1 et 20, et n et o=0.

18. Utilisation de dérivés de 6H-dibenzo[c,e][1,2]-oxaphosphorine-6-oxide, qui ont été produits selon un procédé selon l'une des revendications 1 à 13, à titre d'agent ignifuge pour des polymères et des produits fabriqués à partir de ceux-ci.

19. Utilisation selon la revendication 18, à titre d'agent ignifuge pour des polyesters, des polyamides, des polycarbonates, des polystyrènes, des polyéthylènes, des polypropylènes, des résines phénoliques et époxy.

20. Oligomères de l'acide 1,3,5-tris(2-hydroxyéthyle) cyanurique de la formule (IV) 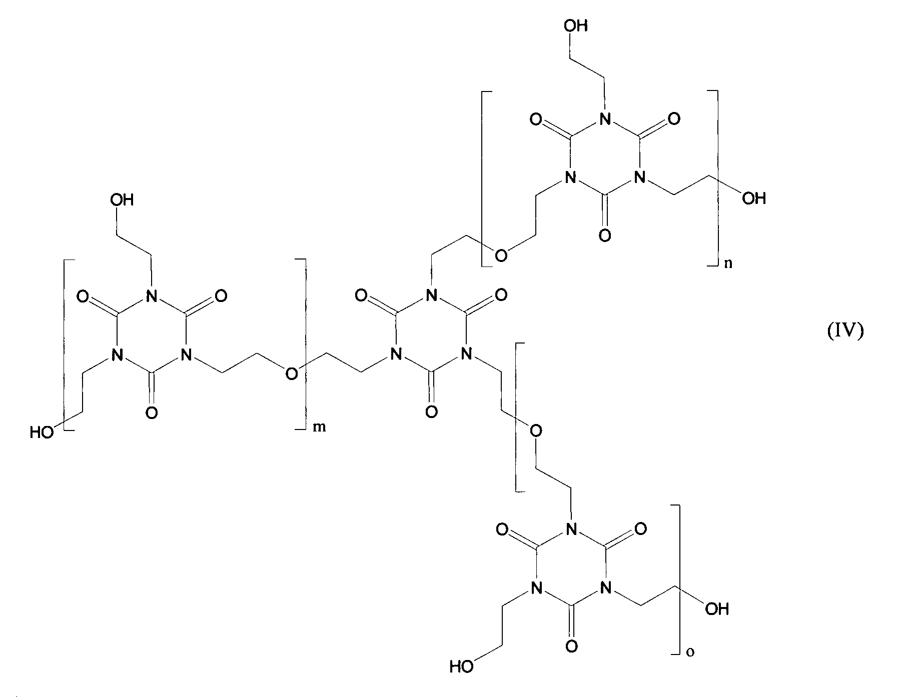 dans laquelle m est un nombre entier entre 1 et 20, et n et o sont des nombres entiers entre 0 et 20.
1. Oligomères selon la revendication 20, présentant la formule (IVa) 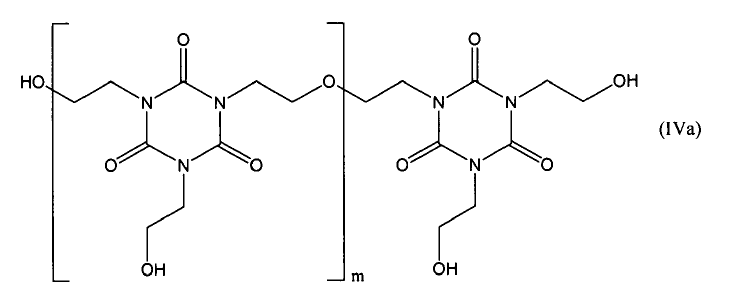 dont la structure dérive de la formule (IV) dans laquelle m est un nombre entier entre 1 et 20, et n et o = 0.
